# EUROPEAN PATENT SPECIFICATION

(11) **EP 0 416 673 B1**
(45) Date of publication and mention of the grant of the patent: **15.11.1995**
(21) Application number: 90202108.8
(22) Date of filing: 02.08.1990
(51) Int. Cl.: C12N 15/62, C12N 15/48, C12N 15/71, C12P 21/02, G01N 33/68

(54) **Method for the expression of heterologous proteins produced in fused form in E. coli, use thereof, expression vectors and recombinant strains**
Verfahren zur Expression von, als Fusionsproteine produzierten, heterologen Proteinen in E. coli, deren Verwendungen, Expressionsvektoren und rekombinante Stämme
Expression de protéines étrangères à partir de protéines de fusion exprimées par E. coli, leurs utilisations, vecteurs d'expression et souches recombinantes

(30) Priority: 03.08.1989 CU 14989
(43) Date of publication of application: 13.03.1991
(73) Proprietor: CENTRO DE INGENIERIA GENETICA Y BIOTECNOLOGIA, Havana (CU)
(72) Inventor: Novoa Pérez, Lidia Inés, Playa, Ciudad de la Habana (CU); Machado Lahera, Jorge A., Playa, Ciudad de la Habana (CU); Fernández Maso, Julio Raul, Nuevo Vedado, Ciudad de la Habana (CU); Benitez Fuentes, Jesús V., Playa, Ciudad de la Habana (CU); Narciandi Diaz, Ramon Emilio, Ave 391 No.18218, Boyeros, Ciudad de la Habana (CU); Rodriquez Reinoso, José Luis, La Lisa, Ciudad de la Habana (CU); Estrada Garcia, Mario Pablo, Playa, Ciudad de la Habana (CU); Garcia Suarez, José, Plaza, Ciudad de la Habana (CU); Herrera Martinez, Luis Saturnino, Playa, Ciudad de la Habana (CU)
(74) Representative: Smulders, Theodorus A.H.J., Ir.

(56) References cited:
- EP-A- 0 227 169
- EP-A- 0 227 938
- EP-A- 0 229 998
- EP-A- 0 345 792
- INDIAN JOURNAL OF BIOCHEMISTRY AND BIOPHYSICS, vol. 25, December 1988, pages504-509; S.G. DEVARE et al.: "Genes of human immunodeficiency virus, type I(HIV-I), their expression in Escherichia coli, and their utility in diagnosisof virus infection"

## Description

The present invention relates to the field of biotechnology and recombinant DNA techniques and in particular to a method for the expression of heterologous proteins synthesized in fused and insoluble form from recombinant E. coli bacteria.

The utility of recombinant DNA technology for producing proteins of interest, of any origin in E. coli, has been extensively demonstrated. For this, a large number of vectors have been developed, although new variants are still necessary owing to the fact that each gene to be cloned and expressed represents an individual case (Denhardt, D.T. and Colasanti, J., Vectors, Butterworths, Stoneham, MA, pp. 179-204, 1987 and Lukacsovich, T. et al., Journal of Biotechnology, 13, 243-250, 1990).

Many eukaryotic polypeptides of clinical or industrial interest, the natural availability of which is scarce, have been obtained by cloning and expression of the genes which code for them in Escherichia coli.

An important problem associated with the production of recombinant proteins in microorganisms is degradation of the product by the host system's own proteases. The stability of the protein can be influenced by different factors such as location of the gene product (Talmadge K. and Gilbert W., Proc. Natl. Acad. Sci. USA 79, 1830-1833, 1982; Moks T. et al., Biochemistry 26, 5239-5244, 1987), selection of the host strain (Buell G. et al., Nucleic Acids Res. 13, 1923-1938, 1985; Bishai W.R. et al., J. Bacteriol. 169, 5140-5151, 1987; Grodberg J. and Dunn, J.J., Bacteriol. 170, 1245-1253, 1988) as well as the conditions of subsequent cultivation and purification (Kitano, K. et al., J. Biotechnol. 5, 77-86, 1987).

Eukaryotic genes cloned in phase with bacterial or synthetic nucleic acid sequences can be expressed as hybrid products in the cellular cytoplasm. Transcription from bacterial promoters as well as translation thereof yields fusion proteins which include bacterial or synthetic polypeptide sequences in addition to the eukaryotic polypeptides (Marston, F.A.O., Biochem. J. 240, 1-12, 1986).

Intracellular synthesis of a fusion protein by expression of a heterologous gene of interest fused to a well-expressed host gene, is a valid means of obtaining high levels of expression of a heterologous protein as well as an increase in stability of the product obtained (Itakura, K. et al., Science, 198, 1056-1063, 1977).

One of the systems used more for this purpose has been to obtain proteins fused to the beta-galactosidase from E. coli (Itakura, K. et al., Science, 198, 1056-1063, 1977). However, the main disadvantage of this system is the large size of this protein, on account of which the desired peptide represents only a small portion of the total hybrid protein (Flores, N. et al., Appl. Microbiol. Biotechnol. 25, 267-271, 1986; Goeddel, D.V. et al., PNAS USA, 76: 106-110).

German patent no. 35 41 856 A1 (Hoechst AG) reports on the possibility of using a stabiliser peptide consisting of at least the first 95 amino acids of the N-terminal end of the human protein interleukine-2 to obtain fusion proteins in insoluble form synthesized in E. coli, with a view to expressing eukaryotic peptides such as proinsulin and hirudine, without reference to the levels of expression reached with this system. In this patent are also included in the genetic construction particular sequences for cleavage of the end product with a view to separating the protein of interest from the stabiliser peptide.

The production of viral proteins by genetic engineering is of great interest for the development of methods of diagnosis and vaccine preparations, above all because of the purity of the resulting products as well as the elimination of manipulation of the active pathogenic agent. In the field of diagnosis, these products are of great importance in early detection of antibodies to these organisms, high specificity and sensitivity in said systems being achieved.

In particular, in the case of human retroviruses, it is necessary to develop highly sensitive systems for the detection of antibodies on the basis of very pure antigens, avoiding any loss of specificity which would invalidate the use of them. These organisms cause various immunological changes, depending on the particular subgroup to which the viral agent belongs, and also due to its trophism for T-lymphocyte cells, being able to cause abnormal proliferation or impaired functionality of said cells (leukaemia) or a depletion of the cell population (immunosuppression) (Wong-Staal, F. and Gallo, R.C., Nature, 317, 395-403, 1985).

It is therefore necessary to count on efficient systems of expression of the main proteins with antigenic activity belonging to the viruses which cause these diseases, with a view to using them in rapid and precise diagnostic systems, which will make it possible to carry out large-scale epidemiological studies for the detection of antibodies to these viruses during processing of blood samples in banks and thus to prevent the disease from being transmitted by this pathway.

The genes which code for the main proteins with antigenic activity of human immunodeficiency viruses (HIV) have been cloned and expressed in E. coli, both directly and fused to other genes belonging to said host.

Among the proteins expressed in their natural form are peptide 121 of AIDS, which is obtained in insoluble form with levels of expression varying between 5 and 10% of total proteins (Chang, T.W. et al., Biotechnology 3, 905-909, 1985) and protein gag 24 of the same virus which is obtained in soluble form at levels not calculated (Dowbenko, D.J. et al., Proc. Natl. Acad. Sci. USA, 82, 7748-7752, 1985).

In Spanish patent no. 2 000 859 (Syntex) is described a method for the expression of fusion proteins using a vector which contains a DNA gene of the protein TrpLE of E. coli, in which is specifically inserted a DNA sequence of the AIDS virus. In this case, the carboxy-terminal LE region is substituted by a heterologous polypeptide, as a result of which a self-aggregating fusion protein is obtained, purification thereof being simplified in this way. Moreover the vector used contains binding means for three reading frames which facilitates isolation of the protein of interest. In this patent is described the construction of a clone of high expression which produces more than 5% of the total cell protein.

The present invention relates to a method for the expression of heterologous proteins produced in insoluble form in E. coli and in particular fusion proteins which contain a fragment or the whole of a viral protein such as the case of antigenic proteins belonging to human immunodeficiency virus (HIV 1 and 2). For this, there was used a vector which contains a stabiliser sequence wich codes for approximately the first 58 amino acids of the N-terminal end of human interleukine-2 (IL-2), which guarantees high levels of expression of the heterologous genes cloned. This vector further consists of the tryptophan promoter of E. coli (ptrp), the gene for resistance to ampicillin as a selection marker, the terminator of transcription of bacteriophage T4 and restriction sites XbaI, XhoI and BamHI for coupling of the genes which it is desired to express. The present invention therefore also relates to the expression vectors used for cloning and expression of the different antigenic proteins of HIV 1 and 2 in E. coli as well as the recombinant strains obtained, which express levels of said heterologous proteins varying between 20 and 25% of the total proteins produced by them.

In particular, the proteins expressed were the one belonging to the nucleus (gag24) and a fragment of the coat protein (gp41) of virus HIV 1 and a fragment of the transmembraneous protein gp36 of HIV 2. The strains used as hosts for cloning of the genes which code for these proteins were E. coli K-12 HB-101, W-3110 and C-600 respectively.

An innovating feature of the present invention is the use of a stabiliser sequence, which consists of a fragment of the N-terminal end of the gene of human interleukine-2 protein which codes only for the first 58 amino acids of said protein, which is used for the expression of heterologous proteins and in particular the main proteins with antigenic activity of the HIV virus.

The fusion proteins expressed by means of the method described are synthesized in insoluble form, which simplifies the final purification process and makes it more efficient, on account of which proteins which display antigenic activity are obtained, which are used in diagnostic methods for the detection of antibodies to them without the need for cleavage of the stabiliser fragment used in the fusion, the present invention also relating to use of the fusion protein obtained.

### EXAMPLES

### Example 1

For the expression of different heterologous proteins in E. coli, there was constructed the expression vector pFP-15, in which was inserted the sequence which codes for a stabiliser peptide, consisting of the first 58 amino acids belonging to the N-terminal fragment of the protein of human origin, interleukine-2 (IL-2). Said sequence is cloned under the control of the tryptophan promoter of E. coli, said vector further comprising the terminator of bacteriophage T4 as a signal of termination of transcription and the gene for ampicillin resistance as a selection marker.

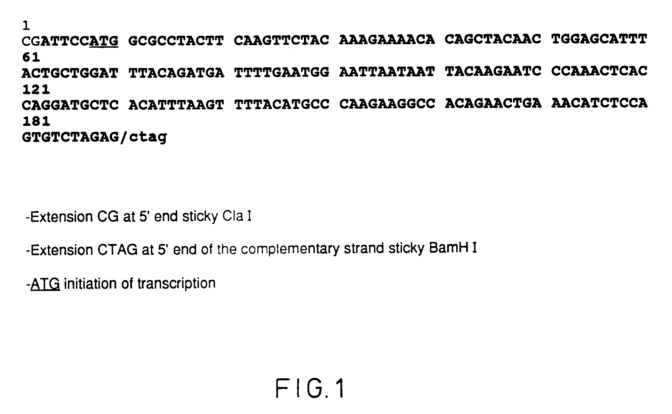

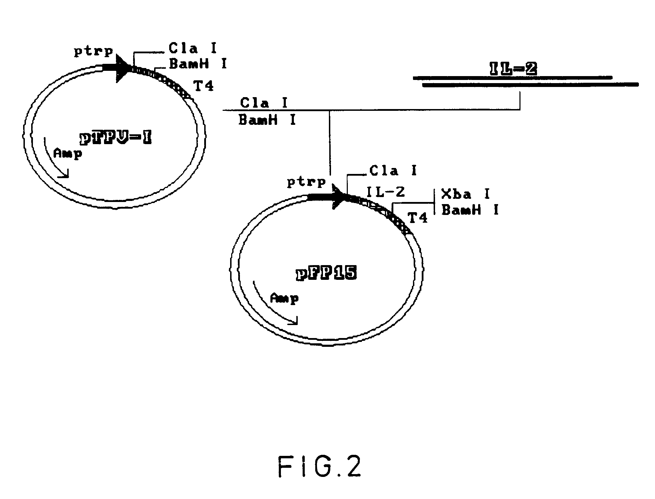

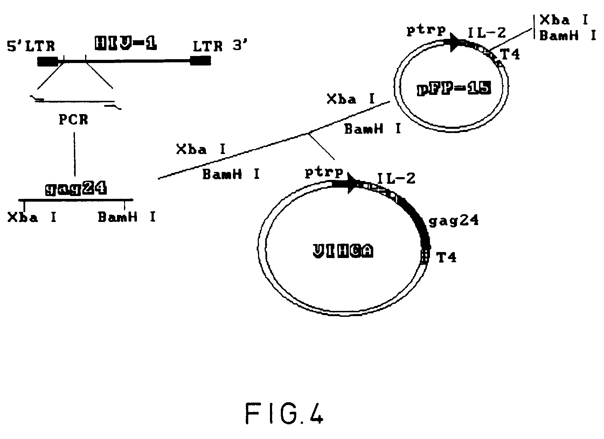

The plasmid vector pFP-15 was constructed by ligation of a synthetic oligonucleotide of 190 bases and its complementary one, which contains the sequence which codes for the first 58 amino acids of the N-terminal end of IL-2 (Fig. 1), the stabiliser sequence, and the vector pTPV-1 (Fig. 2) which carries the tryptophan promoter of Escherichia coli and the terminator of bacteriophage T4. The layout of said construction is shown in Fig. 2.

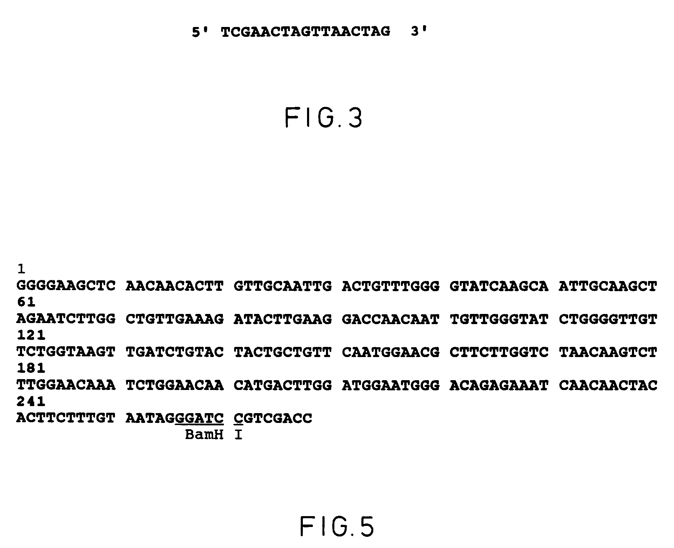

Coupling of the DNA segment which codes for the above stabiliser peptide was verified by DNA sequence analysis according to the description in the literature (Sanger, F. et al., PNAS, USA, 74, 5463-5467, 1977) using an oligonucleotide (Fig. 3) which hybridises with the ptrp promoter and sequence in the direction of the stabiliser (5'-3'). Thus it was possible to check that in all cases the appropriate reading frame was maintained.

### Example 2

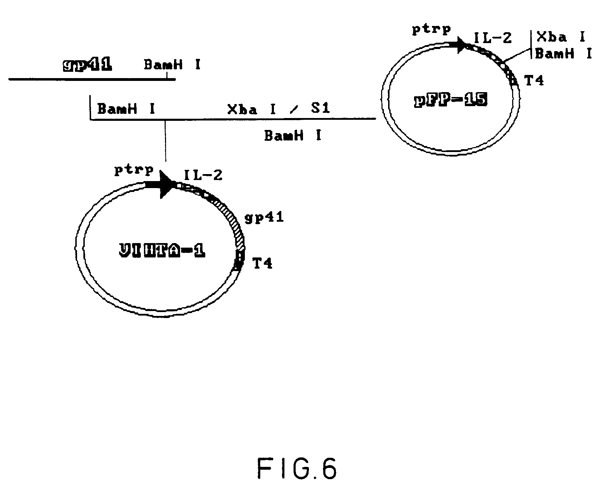

For cloning and expression of the nuclear protein of virus HIV-1 (gag24), the following oligonucleotides were designed: 
5' CAT CTA GAC ATG CAA ATG TTA AAA GAA 3'
3' GT TTA GGT CGA TTG ACT ATC CTA GGC 5'
These oligonucleotides correspond to the 5' and 3' ends respectively of the gene which codes for protein gag24 (Alizon, M. et al., Nature 312, 757-760, 1984). With these oligonucleotides and with the genome of HIV-1 isolated, amplification was carried out by the technique of the polymerase chain reaction (PCR) (Randall, K. et al., Science, USA, 239, 487-491, 1988) of the gene which codes for a fragment of the gag24 gene. This fragment was cut at sites XbaI and BamHI, which were contained in the oligonucleotides used in the PCR, and was ligated to the expression vector pFP-15, XbaI-BamHI being digested, the amplified gene being thus ligated to the segment which codes for the stabiliser peptide, under the tryptophan promoter. The recombinant plasmid obtained, called VIHCA (Fig. 4), was transformed in cells of E. coli strain K-12 HB-101. The transformed colonies were selected for ampicillin resistance in dishes of Luria broth medium (Miller, J.H., Cold Spring Harbor Lab., 1972) supplemented with the antibiotic at 50 ug/ml final concentration, and the recombinants were identified by the technique of hybridisation, using as a radioactive probe (labeled with ³²P) the actual amplified fragment used for cloning. An immunoidentification test was carried out on the positive ones in autoradiography, with serum of infected patients and ¹²⁵I labeled protein A, expression of the protein gag24 being identified by the positivity of these clones in the immunological technique. On these individuals was carried out the Western blot technique (Burnette, W.N., Anal. Bioch., 112, 195-203, 1981), a band of approximately 28,000 daltons being obtained, which corresponds to the length of the stabiliser peptide (approximately 58 amino acids) plus the fragment of the cloned protein gag24 (approximately 180 amino acids).

### Example 3

For cloning and expression of the transmembraneous protein gp41, first of all the synthesis of an oligonucleotide of 269 bases and its complementary one (Fig. 5) was carried out, corresponding to a fragment of said protein belonging to the coat of the virus (Han, B.H. et al., Nature 312, 166-169, 1984). This oligonucleotide was digested with BamHI and ligated to the vector pPF-15 previously cut with XbaI, treated with S1 nuclease and finally digested by BamHI, the desired gene remaining fused to the segment which codes for the stabiliser peptide, under the tryptophan promoter of said vector. The product of this ligation is the vector VIHTA-1 (Fig. 6) with which E. coli strain K-12 W-3110 was transformed. The transformed colonies were selected for ampicillin resistance, and the recombinants were identified by the technique of hybridisation, an immunoidentification test being performed on the positive ones as in the preceding example. Western blot was carried out on one of the individuals which showed expression of the fused protein, a band of approximately 15,000 daltons being obtained, which corresponds to the expected size of the fusion protein, which includes the 58 amino acids of the stabiliser peptide plus 83 amino acids corresponding to the fragment of protein gp41 of HIV-1.

### Example 4

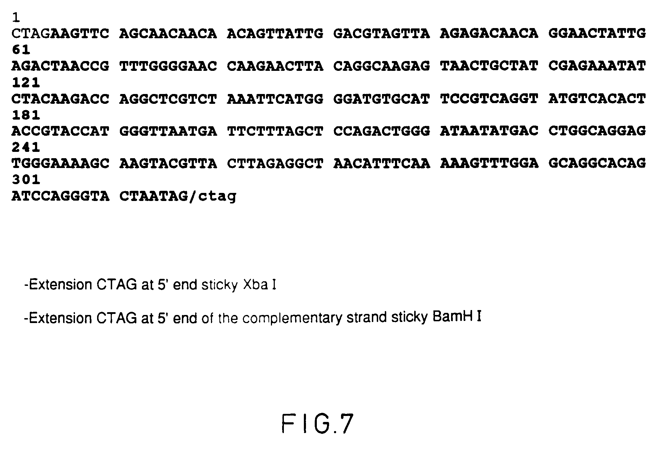

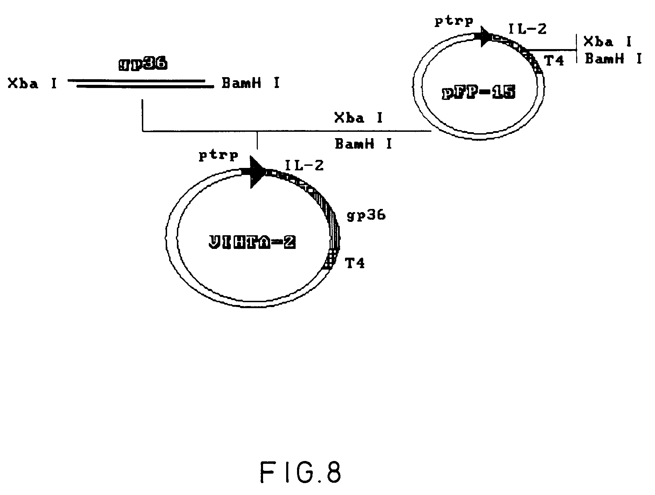

Cloning was carried out of the region representing the gene which codes for expression of the transmembraneous protein of HIV-2, gp36, by the synthesis of an oligonucleotide of 318 bp and its complementary one (Fig. 7) corresponding to a fragment of the protein gp36 of the coat of HIV-2 (Clavel, F. et al., Science 233, 343-346). This DNA segment was ligated to the vector pPF-15 previously cut by XbaI/BamHI, the desired fragment remaining fused to the gene which codes for the stabiliser peptide, under the tryptophan promoter of said vector. The product of this ligation is the vector VIHTA-2 (Fig. 8), which was inserted in E. coli strain K-12 C-600. The transformed colonies were selected for ampicillin resistance, and the recombinants were identified by the technique of hybridisation, an immunoidentification test being performed on the positive ones as in examples 2 and 3.

In all cases, coupling of the DNA segments to the stabiliser was verified by DNA sequence analysis, as reported in the literature (Sanger, F. et al., PNAS, USA, 74, 5463-5467, 1977).

### Example 5

In the case of the fusion proteins gag24-stabiliser peptide and gp41-stabiliser peptide, the respective recombinant strains HB24 and W41 were grown in super broth medium (32 g tryptone and 20 g yeast extract per litre of distilled water) supplemented with FeCl₃ (0.001 mM), MgSO₄ (0.1 mM), M9 salts (Na₂HPO₄ at 6%, KH₂PO₄ at 3%, NaCl at 0.5% and NH₄Cl at 1%) and ampicillin 50 ug/ml.

For the protein gp36-stabiliser peptide, the transformed strain C36 was grown on minimal medium (Maniatis et al., Cold Spring Harbor Lab., USA, 1982) supplemented with casein hydrolysate at 2%, glucose at 2%, 1 mM MgSO₄, 0.1 mM CaCl₂ and ampicillin at the same concentration as in the previous case.

Inoculation of the cultures was carried out at an optical density of 0.05, maintaining them at 37°C for 12 hours, with agitation at 260 rpm, aeration at 1 vvm, finally reaching an optical density of 10 read at 600 nm, induced by depletion of the tryptophan by the addition of indoleacrylic acid (Squires, C.L. et al., Jour. of Mol. Biol., USA, 92, 93-111, 1975) two hours after the start of fermentation. The cells obtained are collected by centrifuging and stored at -20°C to be used subsequently in recovery of the desired product. After ultrasonic rupture of the biomass, levels of expression of 20-25% of the total protein are ascertained by SDS-PAGE electrophoresis of proteins (Laemmli, Nature, UK, 227, 680-685, 1970) and analysis of the series on a SCANNER 65 300, USA.

### STRAIN DEPOSITS

The E. coli HB24 [pVIHCA] strain, based on the E. coli strain K-12 HB-101 and containing the plasmid pVIHCA, was deposited on July 11, 1990, with the Centraalbureau voor Schimmelcultures (CBS), Baarn, The Netherlands, and obtained deposit number CBS307.90.
The E. coli W41 [pVIHTA-1] strain, based on the E. coli strain K-12 W-3110 and containing the plasmid pVIHTA-1, was deposited on July 11, 1990, with the Centraalbureau voor Schimmelcultures (CBS), Baarn, The Netherlands, and obtained deposit number CBS306.90.
The E. coli C36 [pVIHTA-2] strain, based on the E. coli strain K-12 C-600 and containing the plasmid pVIHTA-2, was deposited on July 11, 1990, with the Centraalbureau voor Schimmelcultures (CBS), Baarn, The Netherlands, and obtained deposit number CBS305.90.

## Claims

1. A fusion protein characterized in that it contains at the N-terminus a stabilizer peptide consisting of the first 58 amino acids at the N-terminus of human interleukin-2, which is fused to a heterologous protein.

2. A fusion protein according to claim 1, characterized in that said heterologous protein corresponds to the protein gag24 of HIV-1, protein gp41 of HIV-1, or protein gp36 of HIV-2.

3. A method for expressing a heterologous protein in E. coli in the form of a fusion protein containing a stabilizer peptide at the N-terminus, wherein said stabilizer peptide consists of an N-terminal fragment of human interleukin-2, characterized in that said stabilizer peptide consists of the first 58 amino acids at the N-terminus of human interleukin-2.

4. A method according to claim 3, characterized in that the amino acid sequence of the stabilizer peptide corresponds to (SEQ ID NO:7):

10 20 30 40 50 58
MAPTSSSTKK TQLQLEHLLL DLQMILNGIN NYKNPKLTRM LTFKFYMPKK
ATELKHLQ.

5. A method according to claim 3, characterized in that the heterologous protein which is expressed corresponds to the nuclear protein (gag24) or the transmembraneous protein (gp41) belonging to human immunodeficiency virus HIV-1 or the transmembraneous protein gp36 belonging to human immunodeficiency virus HIV-2.

6. A vector for use in E. coli, characterized in that it contains a stabilizer sequence coding for the first 58 amino acids at the N-terminus of human interleukin-2 under control of the tryptophan promoter of E. coli, contains the termination signal of bacteriophage T4 and the ampicillin resistance gene, and contains the restriction sites XbaI, BamHI and XhoI for insertion of DNA encoding a heterologous protein which is to be expressed.

7. An expression vector for use in E. coli, characterized in that it is derived from a vector (pFP-15) which contains a stabilizer sequence coding for the first 58 amino acids at the N-terminus of human interleukin-2 under control of the tryptophan promoter of E. coli, contains the termination signal of bacteriophage T4 and the ampicillin resistance gene, and contains the restriction sites XbaI, BamHI and XhoI for insertion of DNA encoding a heterologous protein which is to be expressed, by inserting in one of said restriction sites a gene sequence coding for protein gag24 of HIV-1 (vector VIHCA), an 83 aa fragment of protein gp41 of HIV-1 (vector VIHTA-1), or a 106 aa fragment of protein gp36 of HIV-2 (vector VIHTA-2).

8. A recombinant E. coli strain, characterized in that it is obtained as a result of transformation of E. coli strain K-12 HB-101, W-3110 or C-600 with the vector VIHCA, VIHTA-1 or VIHTA-2 respectively and that it expresses high levels of the respective antigenic HIV protein in insoluble form.

9. Use of a fusion protein according to claim 1 or 2 in an in vitro diagnostic method for the detection of human or animal antibodies.

## Patentansprüche

1. Fusionsprotein, dadurch gekennzeichnet, daß es am N-Terminus ein Stabilisatorpeptid enthält, bestehend aus den ersten 58 Aminosäuren am N-Terminus von Human-Interleukin-2, das zu einem heterologen Protein fusioniert wird.

2. Fusionsprotein nach Anspruch 1, dadurch gekennzeichnet, daß das heterologe Protein dem Protein gag24 von HIV-1, dem Protein gp41 von HIV-1 oder dem Protein gp36 von HIV-2 entspricht.

3. Verfahren zur Expression eines heterologen Proteins in E.coli in Form eines Fusionproteins, das ein Stabilisatorpeptid am N-Terminus enthält, wobei das Stabilisatorpeptid aus einem N-terminalen Fragment von Human-Interleukin-2 besteht, dadurch gekennzeichnet, daß das Stabilisatorpeptid aus den ersten 58 Aminosäuren am N-Terminus von Human-Interleukin-2 besteht.

4. Verfahren nach Anspruch 3, dadurch gekennzeichnet, daß die Aminosäuresequenz des Stabilisatorpeptids (SEQ ID NO:7): 
10 20 30 40 50 58
MAPTSSSTKK TQLQLEHLLL DLQMILNGIN NYKNPKLTRM LTFKFYMPKK ATELKHLQ
 entspricht.

5. Verfahren nach Anspruch 3, dadurch gekennzeichnet, daß das hetereologe Protein, das zur Expression gebracht wird, dem Nuklearprotein (gag24) oder dem Transmembran-Protein (gp41), gehörend zum "Human Immunodeficiency Virus" HIV-1, oder dem Transmembran-Protein (gp36), gehörend zum "Human Immunodeficiency Virus" HIV-2, entspricht.

6. Vektor zur Verwendung in E. coli, dadurch gekennzeichnet, daß er eine Stabilisatorsequenz enthält, die für die ersten 58 Aminosäuren am N-Terminus von Human-Interleukin-2 unter Kontrolle des Tryptophan-Promotors von E.coli codiert, das Beendigungssignal des Bakteriophagen T4 und das Ampicillin-Resistenzgen enthält und die Restriktionsstellen XbaI, BamHI und XhoI für die Insertion der DNA enthält, die ein heterologes Protein codiert, das zur Expression zu bringen ist.

7. Expressionsvektor zur Verwendung in E. coli, dadurch gekennzeichnet, daß er von einem Vektor (pFP-15) abgeleitet wird, der eine Stabilisatorsequenz enthält, die für die ersten 58 Aminosäuren am N-Terminus von Human-Interleukin-2 unter Kontrolle des Tryptophan-Promotors von E.coli codiert, das Beendigungssignal des Bakteriophagen T4 und das Ampicillin-Resistenzgen enthält und die Restriktionsstellen XbaI, BamHI und XhoI für die Insertion der DNA enthält, die ein heterologes Protein codiert, das zur Expression zu bringen ist, durch Insertion in eine der genannten Restriktionsstellen einer Gensequenz, die für Protein gag24 von HIV-1 (Vektor VIHCA), ein 83 Aminosäuren-Fragment des Proteins gp41 von HIV-1 (Vektor VIHTA-1) oder ein 106 Aminosäuren-Fragment des Proteins gp36 von HIV-2 (Vektor VIHTA-2) codiert.

8. Rekombinanter E.coli-Stamm, dadurch gekennzeichnet, daß er als Resultat der Transformierung des E.coli-Stammes K-12 HB-101, W-3110 oder C-600 mit dem Vektor VIHCA, VIHTA-1 bzw. VIHTA-2 erhalten wird und daß er hohe Niveaus des jeweiligen Antigen-HIV-Proteins in unlöslicher Form zur Expression bringt.

9. Verwendung eines Fusionsproteins nach Anspruch 1 oder 2 in einem in vitro diagnostischen Verfahren zur Ermittlung von menschlichen oder tierischen Antikörpern.

## Revendications

1. Protéine de fusion caractérisée en ce qu'elle contient au niveau du terminal N un peptide stabilisateur composé des 58 premiers acides aminés du terminal N de l'interleucine-2 humaine qui est fusionnée en une protéine hétérologue.

2. Protéine de fusion selon la revendication 1, caractérisée en ce que ladite protéine hétérologue correspond à la protéine gag24 de l'HIV-1, la protéine gp41 de l'HIV-1 ou la protéine gp36 de l'HIV-2.

3. Procédé d'expression d'une protéine hétérologue dans l'E.Coli sous la forme d'une protéine de fusion contenant un peptide stabilisateur à hauteur du terminal N, dans lequel ledit peptide stabilisateur se compose d'un fragment du terminal N de l'interleucine-2 humaine, caractérisé en ce que ledit peptide stabilisateur se compose des 58 premiers acides aminés du terminal N de l'interleucine-2 humaine.

4. Procédé selon la revendication 3, caractérisé en ce que la séquence d'acides aminés du peptide stabilisateur correspond à la séquence (SEQ ID NO:7):

10 20 30 40 50 58
MAPTSSSTKK TQLQLEHLLL DLQMILNGIN NYKNPKLTRM FKFYMPKK ATELKHLQ.

5. Procédé selon la revendication 3, caractérisé en ce que la protéine hétérologue qui est exprimée correspond à la protéine nucléaire (gag24) ou la protéine transmembraneuse (gp41) appartenant au virus d'immunodéficience humaine HIV-1 ou la protéine transmembraneuse gp36 appartenant au virus d'immunodéficience humaine HIV-2.

6. Vecteur à utiliser dans l'E. Coli, caractérisé en ce qu'il contient une séquence stabilisatrice codant les 58 premiers acides aminés à hauteur du terminal M de l'interleucine-2 humaine sous le contrôle d'un promoteur de tryptophane d'E.Coli, contient le signal de terminaison du bactériophage D4 et le gène de résistance à l'ampicilline et contient les sites de restriction XbaI, BamHI et XhoI pour l'insertion de l'ADM codant une protéine hétérologue qui doit être exprimée.

7. Vecteur d'expression à utiliser dans l'E. Coli, caractérisé en ce qu'il est dérivé d'un vecteur (pFP-15) qui contient une séquence stabilisatrice codant les 58 premiers acides aminés à hauteur du terminal N de l'interleucine-2 humaine sous le contrôle du promoteur de tryptophane de l'E.Coli, contient le signal de terminaison du bactériophage T4 et le gène de résistance à l'ampicilline et contient les sites de restriction XbaI, BamHI et XhoI pour l'insertion de l'ADN codant une protéine hétérologue qui doit être exprimée en insérant dans un desdits sites de restriction une séquence de gènes codant la protéine gag24 de l'HIV-1 (vecteur VIHCA), un fragment de protéine gp41 de l'HIV-1 (vecteur VIHTA-1) ou un fragment de protéine gp36 de l'HIV-2 (vecteur VIHTA-2).

8. Souche d'E.Coli recombinante, caractérisée en ce qu'elle est obtenue à la suite de la transformation d'une souche d'E. coli K-12 HB-101, W-3110 ou C-600 avec, respectivement, le vecteur VIHCA, VIHTA-1 ou VIHTA-2 et en ce qu'elle exprime des taux élevés de la protéine HIV antigène respective sous forme insoluble.

9. Utilisation d'une protéine de fusion selon la revendication 1 ou 2 dans un procédé de diagnostic in-vitro pour la détection d'anticorps humains ou animaux.
